(19) Europäisches Patentamt / European Patent Office / Office européen des brevets

(11) **EP 3 967 211 B1**

(12) **EUROPEAN PATENT SPECIFICATION**

(45) Date of publication and mention of the grant of the patent:
**05.11.2025 Bulletin 2025/45**

(21) Application number: **20801711.1**

(22) Date of filing: **09.04.2020**

(51) International Patent Classification (IPC):
*A61B 3/028* (2006.01)  *A61B 3/04* (2006.01)
*A61B 3/00* (2006.01)  *G02C 7/02* (2006.01)
*A61B 3/103* (2006.01)  *A61B 3/11* (2006.01)

(52) Cooperative Patent Classification (CPC):
**A61B 3/028; A61B 3/0008; A61B 3/04; A61B 3/103; A61B 3/111; A61B 3/112**

(86) International application number:
**PCT/JP2020/015979**

(87) International publication number:
**WO 2020/226023 (12.11.2020 Gazette 2020/46)**

(54) **OPTOMETRIC DEVICE**

OPTOMETRISCHE VORRICHTUNG

DISPOSITIF OPTOMÉTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.05.2019 JP 2019089332**

(43) Date of publication of application:
**16.03.2022 Bulletin 2022/11**

(73) Proprietor: **Nidek Co., Ltd.**
**Gamagori-shi,**
**Aichi, 443-0038 (JP)**

(72) Inventors:
• **TAKII Michihiro**
  **Gamagori-shi Aichi 443-0038 (JP)**
• **TACHIBANA Sasagu**
  **Gamagori-shi Aichi 443-0038 (JP)**

(74) Representative: **Zimmermann, Tankred Klaus et al**
  **Schoppe, Zimmermann, Stöckeler Zinkler, Schenk & Partner mbB**
  **Patentanwälte**
  **Radlkoferstrasse 2**
  **81373 München (DE)**

(56) References cited:
| | |
|---|---|
| EP-A1- 2 949 266 | WO-A1-2017/002846 |
| WO-A1-2017/002846 | JP-A- 2005 342 186 |
| JP-A- 2017 184 790 | JP-A- 2017 213 125 |
| JP-A- 2018 171 229 | JP-A- H1 033 479 |
| US-A- 6 048 064 | US-A1- 2004 027 501 |
| US-A1- 2018 136 486 | |

**Description**

TECHNICAL FIELD

The present disclosure relates to an optometry apparatus that examines the optical property of an examinee's eye.

BACKGROUND ART

An apparatus where optical members (for example, a spherical lens and a cylindrical lens) are placed in front of the eye of an examinee and a target is presented via the optical members to measure the optical property of the examinee's eye is known as an optometry apparatus (refer to Patent Literature 1).

Patent Literature 2 discloses an ophthalmologic apparatus 1 including a refractive power application unit, a storage unit, and a control unit. The refractive power application unit is configured to be capable of changing refractive power applied to a subject's eye. The storage unit stores at least a measurement value of eye refractive power obtained by a measurement performed for the subject's eye in the past. The control unit controls the refractive power application unit to selectively apply the measurement value and one or more refractive values different from the measurement value to the subject's eye in response to the input of an instruction to change refractive power applied to the subject's eye.

Patent Literature 3 discloses a wearable ophthalmic device including an outward facing head-mounted light field camera to receive light from a user's surroundings and to generate numerical light field image data. The device may also include a light field processor to access the numerical light field image data, to obtain an optical prescription for an eye of the user, and to computationally introduce an amount of positive or negative optical power to the numerical light field image data based on the optical prescription to generate modified numerical light field image data. The device may also include a head-mounted light field display to generate a physical light field corresponding to the modified numerical light field image data.

Patent Literature 3 discloses an optometry apparatus. Perception data of the examined eye are gained by means of a perception refractive power measure system. Front glasses data through a lens meter are input by controlling the switch of an input switch panel. In a memory is stored a program for estimating a prescription degree by adjusting a correction degree corresponding the perception data based on the input data. A control unit starts an operation to gain an estimated prescription degree according to the program on receiving the input program action signal of a program switch. Obtained results are displayed on a monitor.

CITATION LIST

PATENT LITERATURE

PATENT LITERATURE 1: JP-A-5-176893

PATENT LITERATURE 2: EP 2 949 266 A1

PATENT LITERATURE 3: US 2018/136486 A1

PATENT LITERATURE 4: JP H10 33479 A

SUMMARY OF THE INVENTION

A known optometry apparatus has not been able to measure the amount of diopter power required to be further added to a refraction corrective device (such as spectacles or contact lenses) that is currently worn by an examinee. Document EP 2 949 266 A1, in particular, describes a standard optometry apparatus that examines the optical property of an examinee's eye, the optometry apparatus comprising: a light projecting optical system configured to project target light flux toward the examinee's eye; a corrective optical system placed in an optical path of the light projecting optical system, the corrective optical system being configured to change the optical property of the target light flux; an information acquisition means configured to acquire information on diopter power of a refraction corrective device worn by the examinee; and a control means configured to control the corrective optical system.

A technical issue of the present disclosure is to provide an optometry apparatus that calculates a more correct spectacle prescription value for an examinee who wears a refraction corrective device with wrong diopter power, considering the problem of the known technology.

The present disclosure is characterized by the following configuration:

An optometry apparatus according to claim 1.

According to the present disclosure, it is possible to calculate a more correct spectacle prescription value for an examinee who wears a refraction corrective device with wrong diopter power.

BRIEF DESCRIPTION OF THE DRAWINGS

Fig. 1 is an external view of an optometry apparatus.
Fig. 2 is a diagram illustrating a measuring unit.
Fig. 3 is a schematic configuration diagram of the inside of the optometry apparatus as viewed from the front.
Fig. 4 is a schematic configuration diagram of the inside of the optometry apparatus as viewed from the side.
Fig. 5 is a schematic configuration diagram of the inside of the optometry apparatus as viewed from above.
Fig. 6 is a diagram illustrating a control system of the optometry apparatus.
Fig. 7 is a flowchart illustrating control operations of the optometry apparatus.

DESCRIPTION OF THE EMBODIMENTS

<Embodiments>

An optometry apparatus according to the present disclosure is described hereinafter. An optometry apparatus according to the embodiment (for example, an optometry apparatus 1) examines the optical property of an examinee's eye. The optometry apparatus includes, for example, a light projecting optical system (for example, a light projecting optical system 30), a corrective optical system (for example, a corrective optical system 60), and an information acquirer (for example, a controller 70), and a controller (for example, the controller 70).

<Light Projecting Optical System>

The light projecting optical system projects target light flux toward the examinee's eye. The corrective optical system is placed in an optical path of the light projecting optical system and in an apparatus body to change the optical property of the target light flux. The light projecting optical system may include a target presenting means for projecting the target light flux toward the examinee's eye. For example, a display (for example, a display 31) may be used as the target presenting means. The display may be, for example, a liquid crystal on silicon (LCOS), a liquid crystal display (LCD), or organic electro luminescence (EL). Moreover, for example, a light source and a digital micromirror device (DMD), or a visible light source and target plate for presenting a target may be used as the target presenting means. The light projecting optical system may include at least one optical member that guides, to the examinee's eye, target light flux that is emitted from the target presenting means.

In the embodiment, the optometry apparatus may not necessarily include the light projecting optical system, and it may be configured in such a manner as to provide an apparatus including a light projecting optical system, separately from the optometry apparatus. In other words, the optometry apparatus may be configured, including at least the corrective optical system described below.

<Corrective Optical system>

The optometry apparatus includes, for example, the corrective optical system. The corrective optical system is placed in the optical path of the light projecting optical system, and changes the optical property of the target light flux. The optical property of the target light flux may be at least any of, for example, the spherical power, cylindrical power, and astigmatic axial angle of the target light flux.

The corrective optical system is simply required to be configured in such a manner as to be capable of changing the optical property of the target light flux. For example, the corrective optical system may be configured in such a manner as to be capable of changing the optical property of the target light flux by controlling an optical element. The optical element may be at least any of, for example, a spherical lens, a cylindrical lens, a cross cylinder lens, a rotary prism, a wavefront modulator, and a varifocal lens. The optical element may be, of course, an optical element different from these optical elements.

Moreover, for example, the corrective optical system may correct the spherical power of the examinee's eye by optically changing a position to present a target (a target presenting distance) to the examinee's eye. **In** this case, it may be configured in such a manner that a target presenting unit is moved in an optical axis direction to optically change the target presenting position. Moreover, in this case, it may be configured in such a manner that an optical element (for example, a spherical lens) placed in the optical path is moved in the optical axis direction to optically change the target presenting position.

The corrective optical system may be configured, including a combination of a configuration that controls the optical element, a configuration that moves the target presenting unit in the optical axis direction, and a configuration that moves the optical element placed in the optical path in the optical axis direction.

Moreover, in the embodiment, the corrective optical system may be configured in such a manner that an optical element is placed between the target presenting means and the optical member for guiding, to the examinee's eye, the target light flux that is emitted from the target presenting unit, which are included in the light projecting optical system, to control the optical element and change the optical property of the target light flux. In other words, the corrective optical system may have a configuration of a phantom lens refractometer (phantom corrective optical system). In this case, the target light flux that is corrected by the corrective optical system is guided to the examinee's eye via the optical member.

<Information Acquirer)

The information acquirer acquires information on the diopter power of a refraction corrective device that is currently worn by the examinee. The information acquirer acquires, for example, the diopter power of the refraction corrective device that is measured by a diopter power measuring instrument such as a lensmeter. The information on the diopter power of the refraction corrective device may not necessarily be the value of the lensmeter. For example, the information on the diopter power of the refraction corrective device may be prescription data of anterior spectacles. The refraction corrective device is, for example, spectacles or contact lenses. The information acquirer may acquire, for example, the distance between the optical axes of spectacles.

<Controller>

The controller controls the corrective optical system and performs an over-refraction on the examinee's eye during wear of the refraction corrective device. The controller calculates a prescription value of a new refraction corrective device on the basis of a refractive error that is acquired as a result of the over-refraction in a subjective examination, and the diopter power information that is acquired by the information acquirer. That is, the optometry apparatus according to the embodiment can easily acquire the amount of diopter power required to be further added to the refraction corrective device that the examinee wears. In other words, the optometry apparatus can acquire an appropriate refraction corrective device prescription value with reference to the current refraction corrective device. Moreover, it is possible to make the examinee to recognize the need of a new refraction corrective device.

Moreover, a refractive error is calculated on the basis of the examination result with the refraction corrective device that is actually worn by the examinee. Therefore, it is possible to prescribe a new refraction corrective device with vision closer to real vision.

The controller may calculate a prescription value by adding the refractive error and the diopter power of the refraction corrective device. The diopter power information that is acquired by the information acquirer may include, for example, the spherical power, cylindrical power, and cylindrical axis of the refraction corrective device. Moreover, the refractive error may include a spherical power error (the error of spherical power), a cylindrical power error (the error of cylindrical power), and a cylindrical axis error (the error of the cylindrical axis). In this case, the controller may calculate prescription values for a new refraction corrective device by adding the spherical power error, the cylindrical power error, and the cylindrical axis error respectively to the spherical power, the cylindrical power, and the cylindrical axis.

The controller may correct the value of the pupil diameter of the examinee's eye on the basis of the information on the diopter power of the refraction corrective device that is acquired by the information acquirer. For example, the magnification varies depending on the diopter power of the refraction corrective device (for example, strong negative power causes the pupil to appear smaller). Accordingly, the controller may correct the value of the pupil diameter by the magnification when calculating the pupil diameter.

The controller may output information for changing the fit of the spectacles in the VD (corneal vertex distance) direction (information on the fit) on the basis of a result of the subjective examination. For example, if the examinee's eye cannot see clearly despite the fact that the subjective examination is performed when wearing spectacles with appropriate lens power, the controller may notify an examiner to check the fit of the spectacles and change VD.

If the refraction corrective device is spectacles with pantoscopic and camber angles adjusted to the examinee, the controller may perform an over-refraction on the examinee's eye during wear of the spectacles. The errors of cylindrical power and cylindrical axis hardly occur when the examination is performed with an actual pantoscopic or camber angle as compared to an examination in unaided vision. A more correct prescription value can be obtained by the over-refraction.

The optometry apparatus may further include an image acquirer (for example, an imaging optical system 100) that acquires an image of the face including the anterior segment of the examinee's eye. In this case, the information acquirer may acquire the distance between the optical axes of the spectacles being the refraction corrective device. The controller may then detect the difference between the interpupillary distance of the examinee that is calculated on the basis of the image acquired by the image acquirer, and the distance between the optical axes of the spectacles. The controller may determine whether or not the distance between the optical axes of the spectacles is appropriate for the interpupillary distance of the examinee, on the basis of the difference.

As another embodiment, the optometry apparatus may include a photographing section (for example, an observing optical system 50), the information acquirer, and the controller. For example, the optometry apparatus may not necessarily include the light projecting optical system and the corrective optical system. For example, the photographing section photographs the examinee's eye and accordingly acquires an anterior segment image. The controller may correct the value of the pupil diameter of the examinee's eye that is acquired on the basis of the anterior segment image, on the basis of the diopter power information. As a result, the optometry apparatus can acquire a more correct pupil diameter.

As another embodiment, the optometry apparatus may include the image acquirer, the information acquirer, and the controller. For example, the optometry apparatus may not necessarily include the light projecting optical system and the corrective optical system. The image acquirer acquires, for example, an image of the face including the anterior segment of the examinee's eye. The information acquirer acquires the distance between the optical axes of the spectacles that the examinee wears. The controller may detect the difference between the interpupillary distance of the examinee that is acquired on the basis of the image, and the optical axis distance. The controller may, for example, determine whether or not the spectacles are appropriate, on the basis of the detection result.

The optometry apparatus according to the embodiment examines the optical property of the examinee's eye. The optical property of the examinee's eye may be at least any of, for example, the eye dioptric power of the examinee's eye (for example, the spherical power, cylindrical power, and astigmatic axial angle of the examinee's eye), contrast sensitivity, and binocular vision function (for example, the amount of heterophoria and the stereopsis function).

<EXAMPLE>

An example being one of typical embodiments is described below.

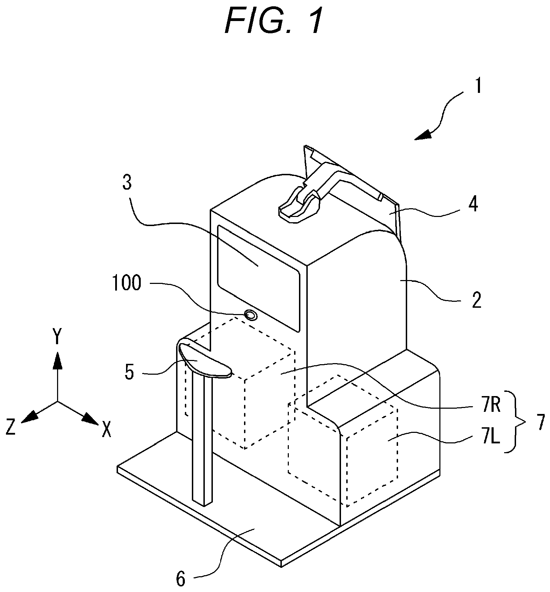

Fig. 1 is an external view of the optometry apparatus 1. The optometry apparatus 1 includes, for example, a housing 2, a presenting window 3, a monitor 4, a chin rest 5, a base 6, and the imaging optical system 100. The housing 2 is fixed to the base 6. A measuring unit 7 described below is provided inside the housing 2. The presenting window 3 is used to present a target to an examinee's eye (examinee's eye E). The monitor 4 displays, for example, a result of measurement of the optical property of the examinee's eye E. The monitor 4 is a display having a touchscreen function. In other words, the monitor 4 functions as an operating device (controller). The monitor 4 may not be a touchscreen. It may be configured in such a manner that the monitor 4 and the operating device are provided separately. In this case, at least any of, for example, a mouse, a joystick, a keyboard, and a mobile terminal may be used as the operating device. A signal in accordance with an operation instruction that is inputted from the monitor 4 is outputted to the controller 70 described below. The chin rest 5 is fixed to the base 6. The chin rest 5 is used to maintain a constant distance between the examinee's eye E and the optometry apparatus 1. The configuration to maintain the constant distance between the examinee's eye E and the optometry apparatus 1 is not limited to the chin rest 5, and, for example, a forehead rest or face rest may be used.

The imaging optical system 100 is used to image the face including the anterior segment of the examinee. The imaging optical system 100 includes an unillustrated imaging device and lens. The imaging optical system 100 images the face including at least one of the examinee's left eye EL and the examinee's right eye ER of the examinee's eyes E, and acquires an image of the face. The imaging of the face by the imaging optical system 100 is controlled by the controller 70 descried below. Moreover, the face image that is acquired by the imaging optical system 100 is analyzed by the controller 70 described below.

<Measuring Unit>

Target light flux from the measuring unit 7 is guided to the examinee's eye E through the presenting window 3. The measuring unit 7 includes a left-eye measuring unit 7L and a right-eye measuring unit 7R. The measuring unit 7 includes a pair of left and right subjective examination parts described below, and a pair of left and right objective examination parts described below. The left-eye measuring unit 7L and the right-eye measuring unit 7R in the example include the same members. At least part of the members included in the left-eye measuring unit 7L and the right-eye measuring unit 7R may, of course, be different.

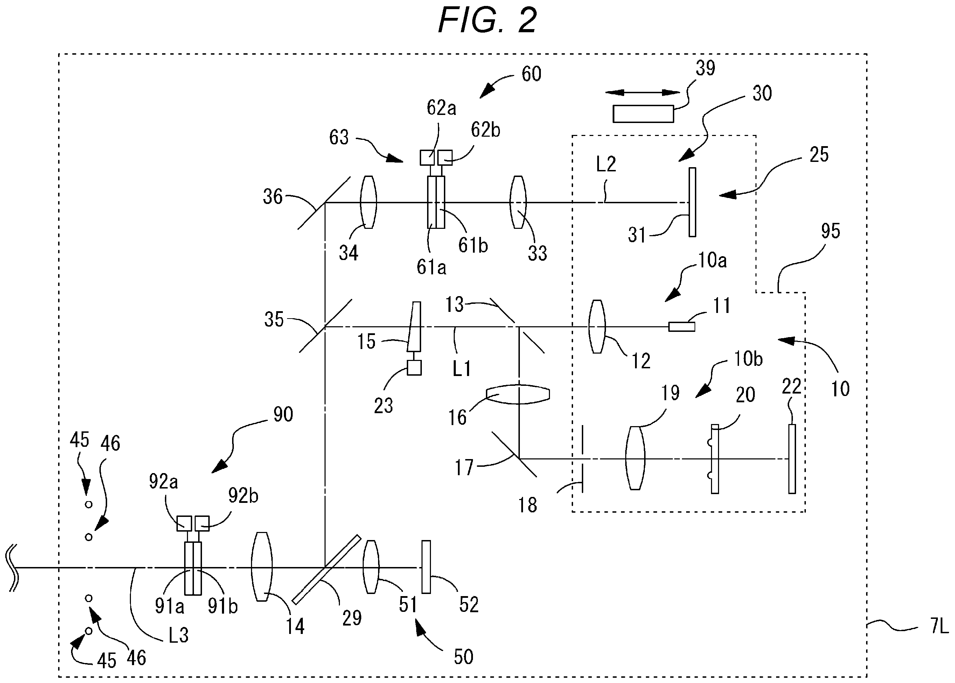

Fig. 2 is a diagram illustrating the measuring unit 7. In Fig. 2, the left-eye measuring unit 7L is taken as an example of the measuring unit 7. The right-eye measuring unit 7R has a configuration similar to that of the left-eye measuring unit 7L, and accordingly, is omitted in Fig. 2. The left-eye measuring unit 7L includes, for example, a subjective examination optical system 25, an objective examination optical system 10, a first target projecting optical system 45, a second target projecting optical system 46, and the observing optical system 50.

<Subjective Examination Optical System>

The subjective examination optical system 25 is used as a part of the configuration of the subjective examination part that subjectively measures the optical property of the examinee's eye E (the details are described below). In the example, the subjective examination part that measures the eye refractive power of the examinee's eye E as the optical property of the examinee's eye E is taken as an example. The optical property of the examinee's eye E may be, for example, contrast sensitivity, or binocular vision function (for example, the amount of heterophoria or the stereopsis function) other than the eye refractive power. The subjective examination optical system 25 includes, for example, the light projecting optical system (target light projecting optical system) 30, the corrective optical system 60, and a correction optical system 90.

<Light Projecting Optical System>

The light projecting optical system 30 projects target light flux toward the examinee's eye E. The light projecting optical system 30 includes, for example, the display 31, a light projection lens 33, a light projection lens 34, a reflective mirror 36, a dichroic mirror 35, a dichroic mirror 29, and an objective lens 14.

Targets (for example, a fixation target and an examination target) are displayed on the display 31. Target light flux that is emitted from the display 31 is projected to the examinee's eye E by way of the optical members in order of the light projection lens 33, the light projection lens 34, the reflective mirror 36, the dichroic mirror 35, the dichroic mirror 29, and the objective lens 14.

<Corrective Optical System>

The corrective optical system 60 is placed in the optical path of the light projecting optical system 30. Moreover, the corrective optical system 60 changes the optical property of the target light flux that is emitted from the display 31. The corrective optical system 60 includes, for example, an astigmatism correction optical system 63 and a drive mechanism 39.

The astigmatism correction optical system 63 is used to correct the cylindrical power and astigmatic axial angle of the examinee's eye E. The astigmatism correction optical system 63 is placed between the light projection lens 33 and the light projection lens 34. The astigmatism correction optical system 63 includes two positive cylindrical lenses 61a and 61b that have equal focal lengths. The cylindrical lenses 61a and 61b rotate independently about an optical axis L2 by driving rotation mechanisms 62a and 62b. In the example, a description has been given, taking the configuration using the cylindrical lenses 61a and 61b as an example of the astigmatism correction optical system 63. However, the astigmatism correction optical system 63 is not limited to this configuration. The astigmatism correction optical system 63 is simply required to be configured in such a manner as to be capable of correcting, for example, the cylindrical power and the astigmatic axial angle. One example of the configuration is a configuration where a corrective lens is put in and out of the optical path of the light projecting optical system 30.

The drive mechanism 39 includes a motor and a sliding mechanism. The drive mechanism 39 moves a drive section 95 described below in the optical axis L2 direction to move the display 31 in the optical axis L2 direction. In the objective examination, the display 31 is moved; accordingly, it is possible to fog the examinee's eye E. In the subjective examination, the display 31 is moved; accordingly, it is possible to optically change the target presenting position (the target presenting distance) to the examinee's eye E and to correct the spherical power of the examinee's eye E. In other words, in the example, a spherical correction optical system that corrects the spherical power of the examinee's eye E by changing the position of the display 31 is configured. The configuration of the spherical correction optical system may be different from that of the example. For example, many optical elements may be placed in the optical path to correct the spherical power. Moreover, for example, a lens may be placed in the optical path, and moved in the optical axis direction to correct the spherical power.

In the example, the corrective optical system that corrects the spherical power, the cylindrical power, and the astigmatic axial angle is exemplified. However, the corrective optical system may correct another optical property (for example, a prism value). The prism value is corrected to appropriately project target light flux to the examinee's eye even if the examinee's eye has heterophoria.

Moreover, in the example, the astigmatism correction optical system 63 that corrects the cylindrical power and the astigmatic axial angle, and the drive mechanism 39 that corrects the spherical power are provided separately. However, the spherical power, the cylindrical power, and the astigmatic axial angle may be corrected by the same configuration. For example, an optical system that modulates the wavefront may correct the spherical power, the cylindrical power, and the astigmatic axial angle. Moreover, a lens disk where a plurality of optical elements (at least any of, for example, spherical lenses, cylindrical lenses, or dispersion prisms) is placed on the same circumference, and an actuator that rotates the lens disk may be used as the corrective optical system. In this case, the lens disk is rotated, and the optical elements are switched to be located on the optical axis L2. Accordingly, various optical properties are corrected. Moreover, an optical element (at least any of, for example, a cylindrical lens, a cross cylinder lens, and a rotary prism) placed on the optical axis L2 may be rotated by an actuator.

<Correction Optical System>

The correction optical system 90 is placed between the objective lens 14 and a deflection mirror 81 (which is described below). The correction optical system 90 is used to correct optical aberration (for example, astigmatism) that occurs in the subjective examination. The correction optical system 90 adjusts the cylindrical power and the astigmatic axial angle to correct astigmatism. The correction optical system 90 includes two positive cylindrical lenses 91a and 91b that have equal focal lengths. The cylindrical lenses 91a and 91b rotate independently about an optical axis L3 by driving rotation mechanisms 92a and 92b. In the example, a description has been given, taking the configuration using the two positive cylindrical lenses 91a and 91b as an example of the correction optical system 90. The correction optical system 90 is not limited to this configuration. The correction optical system 90 is simply required to be configured in such a manner as to be capable of correcting astigmatism. In this case, for example, a correction lens may be put in and out of the optical axis L3.

<Objective Examination Optical System>

The objective examination optical system 10 is used as a part of the configuration of the objective examination part that objectively measures the optical property of the examinee's eye (the details are described below). In the example, a description is given, taking, as an example, the objective examination part that measures the eye refractive power of the examinee's eye E as the optical property of the examinee's eye E. The optical property of the examinee's eye E may be, for example, the ocular axial length or the corneal shape, other than the eye refractive power. The objective examination optical system 10 includes, for example, a projecting optical system 10a, a light receiving optical system 10b, and the correction optical system 90.

The projecting optical system (light projecting optical system) 10a projects a spot-shaped measurement target to the fundus of the examinee's eye E through the pupil center of the examinee's eye E. The projecting optical system 10a includes, for example, a light source 11, a relay lens 12, a hole mirror 13, a prism 15, the dichroic mirror 35, the dichroic mirror 29, and the objective lens 14.

The light source 11 emits measurement light flux. The light source 11 is in a conjugate relation with the fundus of the examinee's eye E. A hole of the hole mirror 13 is in a conjugate relation with the pupil of the examinee's eye E. The prism 15 is a light flux deflecting member. The prism 15 is placed in a position displaced from a position that is conjugate to the pupil of the examinee's eye E to decenter the measurement light flux passing through the prism 15 from an optical axis L1. The prism 15 is driven and rotated about the optical axis L1 by a driver (motor) 23. The dichroic mirror 35 makes an optical path of the objective examination optical system 10 and a below-described optical path of the subjective examination optical system 25 common. In other words, the dichroic mirror 35 makes the optical axis L1 of the objective examination optical system 10 and the optical axis L2 of the subjective examination optical system 25 coaxial. The dichroic mirror 29 is an optical path splitter. The dichroic mirror 29 reflects the measurement light flux by the projecting optical system 10a and the measurement light flux by the subjective examination optical system 25 and guides the measurement light fluxes to the examinee's eye E.

The light receiving optical system 10b extracts fundus reflected light flux that is reflected from the fundus of the examinee's eye E in a ring shape through the periphery of the pupil of the examinee's eye E. The light receiving optical system 10b includes, for example, the objective lens 14, the dichroic mirror 29, the dichroic mirror 35, the prism 15, the hole mirror 13, a relay lens 16, a mirror 17, a light receiving aperture 18, a collimator lens 19, a ring lens 20, and an imaging device 22. The ring lens 20 includes, a lens formed in a ring shape, and a light shielding part being an area excluding the lens that is coated to be shielded from light. The ring lens 20 is in an optically conjugate positional relation with the pupil of the examinee's eye E. The light receiving aperture 18 and the imaging device 22 are in a conjugate relation with the fundus of the examinee's eye E. The output of the imaging device 22 is inputted into the controller 70.

In the example, the light source 11 included in the projecting optical system 10a, the light receiving aperture 18, the collimator lens 19, the ring lens 20, and the imaging device 22 that are included in the light receiving optical system 10b, and the display 31 included in the light projecting optical system 30 are configured in such a manner as to be movable in an integrated manner in the optical axis direction by means of the drive mechanism 39. In other words, the light source 11, the light receiving aperture 18, the collimator lens 19, the ring lens 20, the imaging device 22, and the display 31 are synchronized as the drive section 95, and are moved in an integrated manner by the drive mechanism 39. For example, the moved position to which the drive mechanism 39 moved is detected by an unillustrated potentiometer.

The drive section 95 moves a part of the objective examination optical system 10 in the optical axis direction in such a manner as to cause outer ring light flux to be incident on the imaging device 22 in terms of each meridian direction. In other words, a part of the objective examination optical system 10 is moved in the optical axis L1 direction in accordance with the spherical refractive error (spherical refractive power) of the examinee's eye E to correct the spherical refractive error and optically conjugate the light source 11, the light receiving aperture 18, and the imaging device 22 to the fundus of the examinee's eye E. The hole mirror 13 and the ring lens 20 are placed in such a manner as to be conjugate to the pupil of the examinee's eye E at a constant magnification irrespective of the amount of movement of the drive section 95.

In the above configuration, the measurement light flux that is emitted from the light source 11 forms a spot-shaped point light source image on the fundus of the examinee's eye E by way of the relay lens 12, the hole mirror 13, the prism 15, the dichroic mirror 35, the dichroic mirror 29, and the objective lens 14. At this point in time, the prism 15 that is rotating about the optical axis eccentrically rotates a pupil projected image (projected light flux on the pupil) of the hole of the hole mirror 13 at high speed. The point light source image projected to the fundus is reflected and scattered, exits the examinee's eye E, is condensed by the objective lens 14, and condensed again in the position of the light receiving aperture 18 by way of the dichroic mirror 29, the dichroic mirror 35, the prism 15 that is rotating at high speed, the hole mirror 13, the relay lens 16, and the mirror 17. As a result, a ring-shaped image is formed on the imaging device 22 by the collimator lens 19 and the ring lens 20.

For example, the prism 15 is placed in the common optical path of the projecting optical system 10a and the light receiving optical system 10b. For example, the light flux reflected from the fundus passes through the same prism 15 as the projecting optical system 10a and accordingly, is scanned backwards in the optical system thereafter as if there has been no eccentricity in the projected light flux/reflected light flux (received light flux) on the pupil.

In the example, the configuration of the objective examination part can be changed. For example, the objective examination part may include a configuration that projects a ring-shaped measurement target from the periphery of the pupil to the fundus, extracts the fundus reflected light from the pupil center, and receives a ring-shaped fundus reflected image on the imaging device 22. Moreover, the objective examination part may include a Shack-Hartmann sensor. Moreover, the objective examination part may include a phase difference-type configuration that projects a slit.

<First Target Projecting Optical System and Second Target Projecting Optical System>

In the example, the first target projecting optical system 45 and the second target projecting optical system 46 are placed, for example, between the correction optical system 90 and the deflection mirror 81. The position to place the first target projecting optical system 45 and the second target projecting optical system 46 is not, of course, limited to the above position. The first target projecting optical system 45 and the second target projecting optical system 46 may be included in, for example, a cover of the housing 2. In this case, a configuration where the first target projecting optical system 45 and the second target projecting optical system 46 are placed around the presenting window 3 can be taken as an example.

The first target projecting optical system 45 includes, for example, a ring-shaped infrared light source centered on the optical axis L3. The first target projecting optical system 45 emits, for example, near-infrared light for projecting an alignment target to the cornea of the examinee's eye E. The second target projecting optical system 46 includes, for example, a ring-shaped infrared light source placed in a position different from that of the first target projecting optical system 45. For convenience of description, Fig. 2 schematically illustrates only parts (cross sections) of the ring-shaped infrared light sources in the first target projecting optical system 45 and the second target projecting optical system 46. In the example, the first target projecting optical system 45 projects an infinite alignment target to the cornea of the examinee's eye. Moreover, the second target projecting optical system 46 projects a finite alignment target to the cornea of the examinee's eye. Furthermore, alignment light that is emitted from the second target projecting optical system 46 is also used as anterior segment photographing light for causing the observing optical system 50 to photograph the anterior segment of the examinee's eye. Moreover, the light sources of the first target projecting optical system 45 and the second target projecting optical system 46 are not limited to the ring-shaped light sources, and may be, for example, a plurality of point-shaped, or line-shaped, light sources.

<Observing Optical System>

The observing optical system (imaging optical system) 50 includes, for example, the objective lens 14, the dichroic mirror 29, an imaging lens 51, and an imaging device 52. The dichroic mirror 29 transmits anterior segment observation light and the alignment light. The imaging device 52 has an imaging surface placed in a position that is substantially conjugate to the anterior segment of the examinee's eye E. The output of the imaging device 52 is inputted into the controller 70. As a result, the anterior segment image of the examinee's eye E is the one imaged by the imaging device 52, and the image is displayed on the monitor 4. The observing optical system 50 also acts as an optical system that detects the alignment target images that are formed on the cornea of the examinee's eye E by the first target projecting optical system 45 and the second target projecting optical system 46. The controller 70 detects the positions of the alignment target images.

<Internal Configuration of Optometry Apparatus>

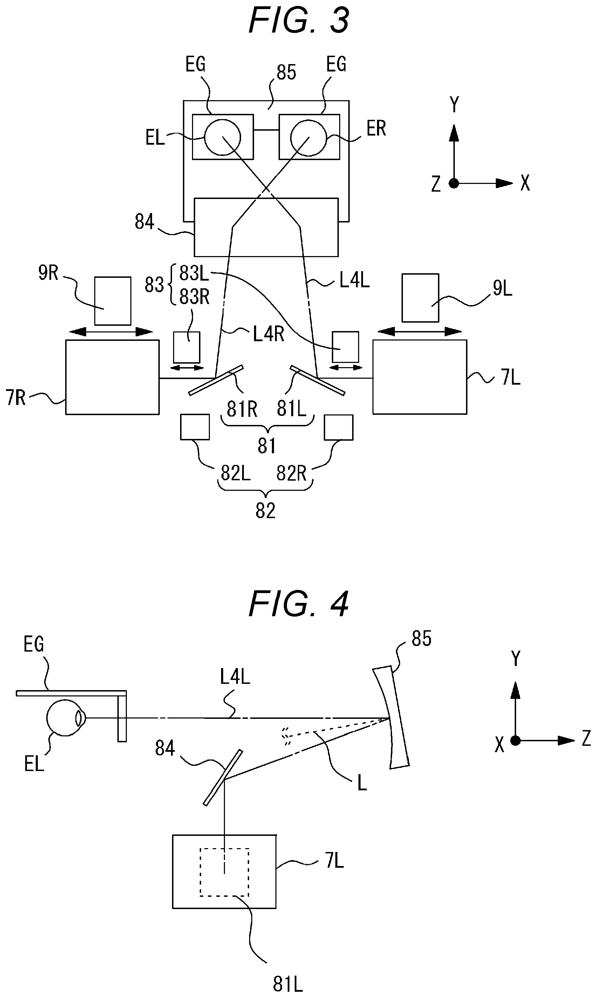

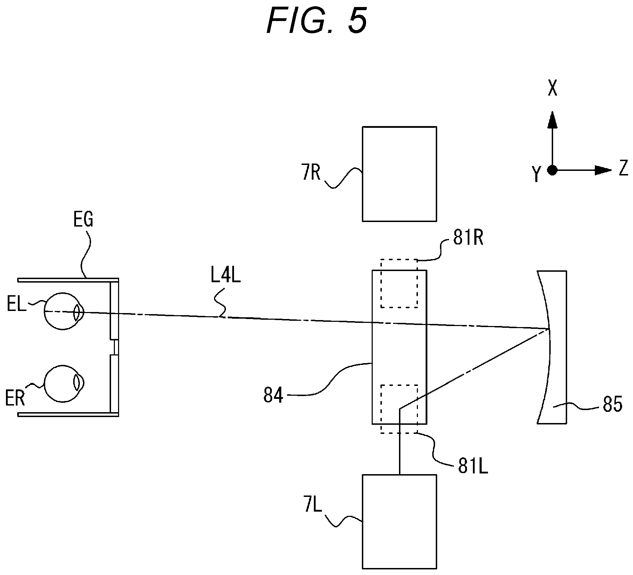

The internal configuration of the optometry apparatus 1 is described below. Fig. 3 is a schematic configuration diagram of the inside of the optometry apparatus 1 according to the example as viewed from the front (in a direction A in Fig. 1). Fig. 4 is a schematic configuration diagram of the inside of the optometry apparatus 1 according to the example as viewed from the side (in a direction B in Fig. 1). Fig. 5 is a schematic configuration diagram of the inside of the optometry apparatus 1 according to the example as viewed from above (in a direction C in Fig. 1). For convenience of description, only an optical axis of the left-eye measuring unit 7L is illustrated in Figs. 4 and 5.

The optometry apparatus 1 includes, for example, the subjective examination part and the objective examination part. The target light flux from the measuring unit 7 may be guided to the examinee's eye E, for example, through an optical path that agrees with an optical axis L of an optical member (for example, a concave mirror 85 described below) in the subjective examination part and the objective examination part. Moreover, for example, the target light flux from the measuring unit 7 may be guided to the examinee's eye E, for example, through an optical path that deviates from the optical axis L of the optical member (for example, the concave mirror 85 described below) in the subjective examination part and the objective examination part. In the example, the optical axis L is, for example, an axis extending toward the center of the sphere of the concave mirror 85. A configuration where the target light flux from the measuring unit 7 passes through the path deviating from the optical axis L of the concave mirror 85 is taken as an example below. In other words, the target light flux from the measuring unit 7 is applied obliquely relative to the optical axis L of the concave mirror 85, and then the reflected light is guided to the examinee's eye E.

The subjective examination part includes, for example, the measuring unit 7, the deflection mirror 81, a drive mechanism 82, a driver 83, a reflective mirror 84, and the concave mirror 85. The subjective examination part is not limited to this configuration. The subjective examination part may have, for example, a configuration without the reflective mirror 84. In this case, the target light flux from the measuring unit 7 may travel via the deflection mirror 81 and then be applied obliquely relative to the optical axis L of the concave mirror 85. Moreover, the subjective examination part may have, for example, a configuration with a half mirror. In this case, the target light flux from the measuring unit 7 may be applied obliquely relative to the optical axis L of the concave mirror 85 via the half mirror, and then the reflected light flux may be guided to the examinee's eye E. Although the concave mirror 85 is placed in the example, it may be configured in such a manner that a convex lens is placed instead of the concave mirror 85.

The objective examination part includes, for example, the measuring unit 7, the deflection mirror 81, the reflective mirror 84, and the concave mirror 85. The objective examination part is not limited to this configuration. The objective examination part may have, for example, a configuration without the reflective mirror 84. In this case, the target light flux from the measuring unit 7 may travel via the deflection mirror 81 and then be applied obliquely relative to the optical axis L of the concave mirror 85. Moreover, the objective examination part may have, for example, a configuration with a half mirror. In this case, the target light flux from the measuring unit 7 may be applied obliquely relative to the optical axis L of the concave mirror 85 via the half mirror, and the reflected light flux may be guided to the examinee's eye E. Although the concave mirror 85 is placed in the example, it may be configured in such a manner that a convex lens is placed instead of the concave mirror 85.

The optometry apparatus 1 includes, for example, a left-eye driver 9L and a right-eye driver 9R, and can move the left-eye measuring unit 7L and the right-eye measuring unit 7R in an X direction. For example, the left-eye measuring unit 7L and the righty-eye measuring unit 7R are moved to change the distance between the deflection mirror 81 and the measuring unit 7 and then change a target light flux presenting position in a Z direction. As a result, the measuring unit 7 can be adjusted in the Z direction in such a manner as to guide, to the examinee's eye E, the target light flux that is corrected by the corrective optical system 60 and then form, on the fundus of the examinee's eye E, an image of the target light flux that is corrected by the corrective optical system 60.

The deflection mirror 81 includes, for example, a deflection mirror 81R for the right eye and a deflection mirror 81L for the left eye that are provided in a pair on the left and right sides, respectively. The deflection mirror 81 is placed, for example, between the corrective optical system 60 and the examinee's eye E. In other words, the corrective optical system 60 according to the example includes left-eye and right-eye corrective optical systems provided in a pair on the left and right sides. The deflection mirror 81L for the left eye is placed between the left-eye corrective optical system and the examinee's left eye EL, and the deflection mirror 81R for the right eye is placed between the right-eye corrective optical system and the examinee's right eye ER. It is preferable to place the deflection mirror 81, for example, in a position that is conjugate to the pupil.

For example, the deflection mirror 81L for the left eye reflects the light flux projected by the left-eye measuring unit 7L, and guides the light flux to the examinee's left eye EL. Moreover, for example, the deflection mirror 81L for the left eye reflects the reflected light reflected from the examinee's left eye EL, and guides the reflected light to the left-eye measuring unit 7L. For example, the deflection mirror 81R for the right eye reflects the light flux projected by the right-eye measuring unit 7R, and guides the light flux to the examinee's right eye ER. Moreover, for example, the deflection mirror 81R for the right eye reflects the reflected light reflected from the examinee's right eye ER, and guides the reflected light to the right-eye measuring unit 7R. In the example, a description has been given, taking the configuration using the deflection mirror 81 as an example of the deflection member that reflects the light flux projected by the measuring unit 7 and guides the light flux to the examinee's eye E. The deflection member is not limited to this configuration. The deflection member is simply required to be a deflection member that reflects the light flux projected by the measuring unit 7 and guides the light flux to the examinee's eye E. Examples of the deflection member include a prism and a lens.

The drive mechanism 82 includes, for example, a motor (driver). The drive mechanism 82 includes, for example, a drive mechanism 82L for driving the deflection mirror 81L for the left eye and a drive mechanism 82R for driving the deflection mirror 81R for the right eye. For example, the drive of the drive mechanism 82 allows the deflection mirror 81 to rotationally move. The drive mechanism 82 rotates the deflection mirror 81, for example, about a rotation axis in a horizontal direction (X direction) and a rotation axis in a vertical direction (Y direction). In other words, the drive mechanism 82 rotates the deflection mirror 81 in the X and Y directions. The deflection mirror 81 may rotate in one of the horizontal and vertical directions.

The driver 83 includes, for example, a motor. The driver 83 includes, for example, a driver 83L for driving the deflection mirror 81L for the left eye and a driver 83R for driving the deflection mirror 81R for the right eye. For example, the drive of the driver 83 allows the deflection mirror 81 to move in the X direction. For example, the deflection mirror 81L for the left eye and the deflection mirror 81R for the right eye are moved, which allows changing the distance between the deflection mirror 81L for the left eye and the deflection mirror 81R for the right eye and changing the distance in the X direction between the left-eye optical path and the right-eye optical path in accordance with the interpupillary distance of the examinee's eye E.

For example, a plurality of the deflection mirrors 81 may be provided in each of the left- and right-eye optical paths. A configuration where two deflection mirrors are provided in each of the left- and right-eye optical paths (for example, two deflection mirrors in the left-eye optical path) is taken as an example. In this case, one of the deflection mirrors may be rotated in the X direction, and the other deflection mirror in the Y direction. For example, the rotational movement of the deflection mirror 81 allows deflecting apparent light flux for forming an image of the corrective optical system 60 in front of the examinee's eye; accordingly, the position to form the image can be optically corrected.

For example, the concave mirror 85 is shared between the right-eye measuring unit 7R and the left-eye measuring unit 7L. For example, the concave mirror 85 is shared between the right-eye optical path including the right-eye corrective optical system and the left-eye optical path including the left-eye corrective optical system. In other words, the concave mirror 85 is placed in a position through which both of the right-eye optical path including the right-eye corrective optical system and the left-eye optical path including the left-eye corrective optical system pass. It may not of course be configured in such a manner that the concave mirror 85 is shared between the right- and left-eye optical paths. In other words, it may be configured in such a manner that a concave mirror is provided to each of the right-eye optical path including the right-eye corrective optical system and the left-eye optical path including the left-eye corrective optical system. For example, the concave mirror 85 guides target light flux passed through the corrective optical system to the examinee's eye E, and forms an image of the target light flux passed through the corrective optical system in front of the examinee's eye E. In the example, a description has been given, taking the configuration using the concave mirror 85 as an example. However, various optical members can be used without being limited to the configuration using the concave mirror 85. For example, a lens or a planar mirror can be used as the optical member.

For example, the concave mirror 85 is used by both of the subjective examination part and the objective examination part. For example, the target light flux that is projected by the subjective examination optical system 25 is projected to the examinee's eye E via the concave mirror 85. For example, the measurement light that is projected by the objective examination optical system 10 is projected to the examinee's eye E via the concave mirror 85. Moreover, for example, the reflected light of the measurement light that is projected by the objective examination optical system 10 is guided to the light receiving optical system 10b of the objective examination optical system 10 via the concave mirror 85. In the example, the configuration where the reflected light of the measurement light by the objective examination optical system 10 is guided to the light receiving optical system 10b of the objective examination optical system 10 via the concave mirror 85 is taken as an example. However, without being limited to this configuration, for example, it may be configured in such a manner that the reflected light of the measurement light by the objective examination optical system 10 does not travel via the concave mirror 85.

More specifically, in the example, for example, an optical axis between the concave mirror 85 and the examinee's eye E in the subjective examination part and an optical axis between the concave mirror 85 and the examinee's eye E in the objective examination part may be configured in such a manner as to be at least coaxial. In the example, for example, the dichroic mirror 35 combines the optical axis L2 of the subjective examination optical system 25 and the optical axis L1 of the objective examination optical system 10 to be coaxial.

<Optical Path of Subjective examination part>

The optical path of the subjective examination part is described below. For example, the subjective examination part uses the concave mirror 85 to reflect target light flux passed through the corrective optical system 60 toward the examinee's eye, guides the target light flux to the examinee's eye E, and forms an image of the target light flux passed through the corrective optical system 60 at a predetermined optical examination distance in front of the examinee's eye E. For example, at this point in time, the target light flux passed through the corrective optical system 60 passes through the optical path deviating from the optical axis L of the concave mirror 85, is incident on the concave mirror 85, is reflected in such a manner as to pass through the optical path deviating from the optical axis L of the concave mirror 85, and is guided to the examinee's eye E. For example, the target that the examinee sees appears farther than an actual distance from the examinee's eye E to the display 31. In other words, the use of the concave mirror 85 allows extending the distance to present a target to the examinee's eye E and presenting the target to the examinee in such a manner that the examinee sees an image of the target light flux in a position at the predetermined examination distance.

A more detailed description is given. The left-eye optical path is taken as an example in the following description. However, the right-eye optical path also has a configuration similar to that of the left-eye optical path. For example, in the subjective measuring unit for the left eye, target light flux that is projected by the display 31 of the left-eye measuring unit 7L enters the astigmatism correction optical system 63 through the light projection lens 33. The target light flux passed through the astigmatism correction optical system 63 enters the correction optical system 90 via the reflective mirror 36, the dichroic mirror 35, the dichroic mirror 29, and the objective lens 14. The target light flux passed through the correction optical system 90 is guided from the left-eye measuring unit 7L toward the deflection mirror 81L for the left eye. The target light flux that is emitted from the left-eye measuring unit 7L and reflected by the deflection mirror 81 for the left eye is reflected by the reflective mirror 84 toward the concave mirror 85. For example, the target light flux that is emitted from the display 31 travels by way of the optical members in this manner, and then reaches the examinee's left eye EL.

Consequently, the target that is corrected by the corrective optical system 60 is formed on the fundus of the examinee's left eye EL with reference to the position of wear of the spectacles (for example, approximately 12 mm from the position of the corneal apex) of the examinee's left eye EL. Therefore, the fact that the astigmatism correction optical system 63 appears to be placed in front of the eye, and the fact that the spherical power is adjusted in front of the eye by a spherical power corrective optical system (in the example, the drive of the drive mechanism 39) are equivalent. The examinee can collimate an image of the target in a natural state via the concave mirror 85. In the example, the right-eye optical path also has a configuration similar to that of the left-eye optical path. It is configured in such a manner that the targets that is corrected by the pair of the left and right corrective optical systems 60 are formed on the fundi of both of the examinee's eyes with reference to the positions of wear of the spectacles (for example, approximately 12 mm from the position of the corneal apex) of the examinee's left eye EL and right eye ER. In this manner, the examinee replies to the examiner while looking directly at the target in natural vision, encourages correction by the corrective optical system 60 until the target is visible at an appropriate level, and subjectively measures the optical property of the examinee's eye on the basis of the corrected value.

<Optical Path of Objective examination part>

Next, the optical path of the objective examination part is described. The left-eye optical path is taken as an example in the following description. However, the right-eye optical path also has a configuration similar to that of the left-eye optical path. For example, in the objective measuring unit for the left eye, the measurement light that is emitted from the light source 11 of the projecting optical system 10a in the objective examination optical system 10 enters the correction optical system 90, passing from the relay lens 12 to the objective lens 14. The measurement light passed through the correction optical system 90 is projected toward the deflection mirror 81L for the left eye by the left-eye measuring unit 7L. The measurement light that is emitted from the left-eye measuring unit 7L and reflected by the deflection mirror 81 for the left eye is reflected toward the concave mirror 85 by the reflective mirror 84. The measurement light that is reflected by the concave mirror passes through the reflective mirror 84, reaches the examinee's left eye EL, and forms a spot-shaped point light source image on the fundus of the examinee's left eye EL. At this point in time, the prism 15 that is rotating about the optical axis eccentrically rotates a pupil projected image (projected light flux on the pupil) of the hole of the hole mirror 13 at high speed.

The light of the point light source image that is formed on the fundus of the examinee's left eye EL is reflected and scattered, exits the examinee's eye E, travels through the optical path where the measurement light passed, is condensed by the objective lens 14, and travels from the dichroic mirror 29 to the dichroic mirror 35 to the prism 15 to the hole mirror 13 to the relay lens 16 to the mirror 17. The reflected light passed the mirror 17 is condensed again on the opening of the light receiving aperture 18, converted by the collimator lens 19 to substantially parallel light flux (if in a state of emmetropia), extracted as ring-shaped light flux by the ring lens 20, and received as a ring image by the imaging device 22. The received ring image is analyzed to enable the objective measurement of the optical property of the examinee's eye E.

<Controller>

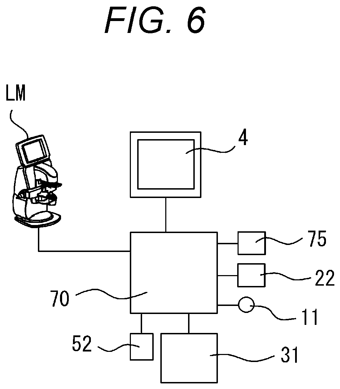

Fig. 6 is a diagram illustrating a control system of the optometry apparatus 1 according to the example. For example, the controller 70 is electrically connected to various members such as the monitor 4, nonvolatile memory 75 (hereinafter referred to as the memory 75) and a lensmeter LM, the light source 11, the imaging device 22, the display 31, and the imaging device 52 that are included in the measuring unit 7. Moreover, the controller 70 is electrically connected to, for example, the driver 9, the drive mechanism 39, the rotation mechanisms 62a and 62b, the driver 83, and unillustrated drivers included respectively in the rotation mechanisms 92a and 92b.

The controller 70 includes, for example, a processor (CPU), RAM, and ROM. The CPU is responsible for, for example, the control of the members of the optometry apparatus 1. The RAM temporarily stores, for example, various kinds of information. For example, various programs for controlling the operation of the optometry apparatus 1, target data for various examinations, and initial values are stored in the ROM. The controller 70 may include a plurality of controllers (that is, a plurality of processors).

The memory 75 is, for example, a non-transitory storage medium that can hold storage contents even if power is shut off. For example, a hard disk drive, flash memory, and a USB flash drive can be used as the memory 75. For example, a control program for controlling the subjective examination part and the objective examination part is stored in the memory 75.

<Control Operations>

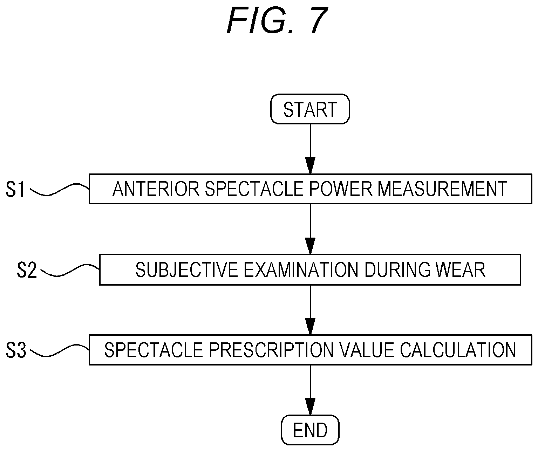

The control operations of the optometry apparatus 1, together with an examination procedure, is described on the basis of Fig. 7.

(S1: Anterior Spectacle Power Measurement)

Before the subjective examination on the optometry apparatus 1, the examiner measures the diopter power of anterior spectacles (spectacles that are currently worn) of the examinee by use of a diopter power measuring instrument such as the lensmeter LM. The lensmeter LM acquires the spherical power, cylindrical power, and cylindrical axis of the spectacle lens. Moreover, the lensmeter LM can also acquire the distance between the optical axes of the spectacles. Moreover, the lensmeter LM can also measure contact lenses. The controller 70 acquires, for example, the diopter power and optical axis distance of the anterior spectacles from the lensmeter LM. In this manner, the controller 70 functions as an information acquirer that acquires information on the diopter power of a refraction corrective device. The diopter power of spectacles may be measured before or after the examination on the optometry apparatus 1. The controller 70 acquires information on the diopter power of the anterior spectacles at any timing after the lensmeter LM measures the diopter power of the spectacles.

<S2: Subjective Examination During Wear>

Next, the subjective examination is performed on the examinee's eye E in a state of wearing the anterior spectacles (in the as-worn state). The examiner presents a fixation target to the examinee's eye E and also projects an alignment target image to the cornea of the examinee's eye E to complete alignment of the examinee's eye E with the measuring unit 7. When the alignment is complete, the controller 70 causes the display 31 to display an examination target in response to the operation of the examiner, and projects the examination target to the examinee's eye E.

For example, the examiner asks the examinee about the orientation of the displayed examination target (for example, the direction of the gap of a Landolt C target), and checks whether or not a diopter power over-refraction for additionally correcting the examinee's eye E in the as-worn state is appropriate, considering the answer from the examinee. If the answer from the examinee is wrong and the diopter power over-refraction is inappropriate, the examiner changes the diopter power and checks again whether or not the diopter power over-refraction is appropriate.

The controller 70 controls at least one of the light projecting optical system 30 and the corrective optical system 60 in the subjective examination optical system 25 in response to an operation signal, and additionally corrects the eye dioptric power of the examinee's eye E in the as-worn state to a predetermined dioptric value (for example, 0 D). For example, the controller 70 may move the display 31 in the optical axis L2 direction, and correct the spherical power of the examinee's eye E. The controller 70 may rotate the cylindrical lenses 61a and 61b about the optical axis L2, and correct at least one of the cylindrical power and astigmatic axial angle of the examinee's eye E.

The examiner repeats a change in the diopter power over-refraction and a check on the vision of the examinee. As a result, the eye dioptric power of the examinee's eye E in the as-worn state is additionally corrected to the predetermined dioptric value, and also the diopter power over-refraction (that is, a refractive error) is acquired.

The objective examination may be performed before the subjective examination. For example, the controller 70 may acquire the optical property (that is, the over-refraction value) of the examinee's eye E in the as-worn state in the objective examination by the objective examination optical system 10. In this case, the controller 70 may additionally correct the examinee's eye E in the as-worn state on the basis of the over-refraction value before the subjective examination.

(S3: Spectacle Prescription Value Calculation)

When the refractive error in the as-worn state is acquired by the subjective examination during wear, the controller 70 calculates correct spectacle prescription values on the basis of the refractive error and the spectacle power that is acquired by the lensmeter LM. Assuming that the diopter power of the anterior spectacles is, for example, S (lens), C (lens), and A (lens), the diopter power of the anterior spectacles can be separated into three components of the Jackson cross cylinder by means of a sin component, a cos component, and spherical equivalent power, and expressed as in equation (1) below.
[Math. 1]

$$J45(lens) = -C(lens)/2 \times \sin(2A(lens))$$
$$J180(lens) = -C(lens)/2 \times \cos(2A(lens)) \qquad (1)$$
$$M(lens) = S(lens) \times C(lens)/2$$

M (lens) is spherical equivalent power, J45 (lens) is cylindrical power in a 45-degree oblique direction, and J180 (lens) is cylindrical power in horizontal and perpendicular directions. Assuming that the diopter power as measured on the optometry apparatus 1 in the as-won state is S (o-ref), C (o-ref), and A (o-ref), it can be expressed as in equation (2) below.
[Math. 2]

$$J45(o-ref) = -C(o-ref)/2 \times \sin(2A(o-ref))$$
$$J180(o-ref) = -C(o-ref)/2 \times \cos(2A(o-ref)) \qquad (2)$$
$$M(o-ref) = S(o-ref) \times C(o-ref)/2$$

At this point in time, more appropriate parameters (S (optim), C(optim), and A (optim)) of the spectacle power are the sum of equations (1) and (2), and can be expressed as in equation (3) below.
[Math. 3]

$$J45(optim) = J45(lens) + J45(o-ref)$$
$$J180(optim) = J180(lens) + J180(o-ref) \qquad (3)$$
$$M(optim) = M(lens) + M(o-ref)$$

On the basis of equation (3), the prescription values for new spectacles can be expressed as in equation (4) below.
[Math. 4]

$$C(optim) = -2\sqrt{\left((J45(optim)) \times 2 + (J180(optim)) \times 2\right)}$$
$$S(optim) = M(optim) - C(optim)/2 \qquad (4)$$
$$A(optim) = 1/2\tan^{-1}(J45(optim)/J180(optim))$$

The controller 70 calculates the prescription values for the new spectacles on the basis of the above equations, and outputs the prescription values. For example, the controller 70 may display the prescription values on the monitor 4, print the prescription values on a printer, or transmit the prescription values to an external apparatus. The examiner makes the new spectacles on the basis of the outputted prescription values.
As described above, the optometry apparatus 1 according to the example performs the subjective examination on an examinee during wear of anterior spectacles, and accordingly can easily acquire the amount of diopter power required to be further added to the anterior spectacles. Moreover, more correct spectacle prescription values can be calculated by means of the result of the subjective examination in the as-worn state and the diopter power of the anterior spectacles. The examination is performed during wear of the anterior spectacles; accordingly, a difference hardly occurs in vision between during the examination and when wearing spectacles that is newly prescribed, and it is possible to reduce discomfort that the examinee feels, as compared to if the examination is performed on the unaided eyes as before. Moreover, the examinee can recognize the need of new spectacles if the examinee's vision is improved by an over-refraction during wear of the anterior spectacles.

As in the above example, the subjective examination is performed during wear of a refraction corrective device, which enables the range of diopter power that can be examined to be extended. Assuming that the measurement range of the S value is, for example, from -15 to +15 D, an examinee with -20 D cannot take the examination in unaided vision. However, if the examination is performed during wear of a refraction corrective device with -10 D, the measurement rage is in effect from -25 D to +5 D; accordingly, the examinee with -20 D can also take the examination. This is valid with regard to the C value.

The optometry apparatus 1 can change the diopter power of a target in the apparatus as in the example. Accordingly, there is no need to physically add, to a spectacle lens, an optical member that changes the dioptric power. As a result, a discrepancy in, for example, VD (corneal vertex distance) is small, and the subjective test can be performed in vision closer to real vision with spectacles on.

Spectacles generally include the pantoscopic angle and the camber angle. The pantoscopic angle is, for example, the angle of the spectacle lens surface relative to the vertical direction. The camber angle is, for example, the angle of the spectacle lens surface relative to the horizontal direction along the bridge of the spectacles. The cylindrical power and the cylindrical axis may change depending on the pantoscopic angle or camber angle. The change is significant especially in spectacles with strong diopter power. For example, if an apparatus where an optical element is placed in a switchable manner in front of the unaided eye of the examinee to perform the subjective examination (what is called a refractor) is used, a measurement is performed without the pantoscopic angle and the camber angle. Accordingly, the vision may be different from real vision during wear of spectacles to be made. In the optometry apparatus according to the example, spectacle prescription values are determined on the basis of a result of the subjective examination in the as-worn state under the influence of the pantoscopic angle and the camber angle. As a result, it is possible to provide spectacles considering vision with the pantoscopic angle and the camber angle as compared to if the examination is performed on the unaided eyes.

The optometry apparatus 1 may measure the pupil diameter of the examinee's eye E. For example, the optometry apparatus 1 may perform an analysis process on an image of the anterior segment of the examinee's eye E that is captured by the observing optical system 50 to acquire the pupil diameter. For example, the controller 70 may acquire the pupil diameter on the basis of the luminance distribution of the anterior segment image.

If the pupil diameter is measured, the size of the pupil diameter is increased or reduced, depending on the spectacle lens power, during wear of spectacles. For example, if the lens has a negative dioptric value, the pupil diameter on the image appears small. If the lens has a positive dioptric value, the pupil diameter on the image appears large. Hence, the controller 70 may correct the pupil diameter on the basis of the spectacle power that is acquired in advance by, for example, the lensmeter LM. For example, the controller 70 may calculate a correction by means of a correction value that is preset for each spectacle lens dioptric value to correct the pupil diameter that is acquired on the basis of the analysis of the anterior segment image. If the spectacle lens has, for example, a negative dioptric value, a correction is made in such a manner as to increase the pupil diameter in accordance with the dioptric value. If the spectacle lens has a positive dioptric value, a correction is made in such a manner as to reduce the pupil diameter in accordance with the dioptric value. As a result, it is possible to acquire a more correct pupil diameter even during wear of a refraction corrective device.

The controller 70 may calculate the pupil diameter on the basis of a face image captured by the imaging optical system 100. Also in this case, the pupil diameter may be corrected in accordance with the spectacle lens power.

If the subjective examination is performed during wear of spectacles with appropriate lens power but the examinee's eye cannot see clearly, the fit of the spectacles may be inappropriate. For example, VD may not be set correctly. In such a case, the controller 70 may notify the examiner that the fit of the spectacles is inappropriate. For example, the controller 70 may notify the amount of discrepancy in VD with respect to a correct value. For example, if spectacles with -10 D are made, the subjective examination is performed, and as a result, a discrepancy of 0.2 D occurs, the controller 70 may display on the monitor 4, for example, a warning to the effect that there is a difference of approximately 2 mm in VD. As a result, the examiner can easily grasp that the fit of the spectacles is inappropriate.

For example, when spectacles with VD = 12 mm are made, if VD is not equal to 12 mm on spectacles that are worn during the subjective examination (for example, if there is a discrepancy in VD on a trial frame), the controller 70 may correct the refractive power of a target by the amount of discrepancy in VD.

The controller 70 may detect a discrepancy between the PD (interpupillary distance) of the examinee and the distance between the optical axes of the spectacle lenses. For example, the controller 70 calculates PD on the basis of an image of the face of the examinee that is captured by the imaging optical system 100. The controller 70 then compares the calculated PD of the examinee and the distance between the optical axes of the spectacles that is measured by the lensmeter LM. If the difference is equal to or greater than a predetermined value, the controller 70 may display on, for example, the monitor 4 information to the effect that the distance between the optical axes of the spectacle lenses is not appropriate. Not only the PD of the examinee and the distance between the optical axes of the spectacles but also an optical axis misalignment in the X and Y directions (up, down, left, and right) between the visual axis of the examinee's eye and the optical axis of the spectacle lens may be detected.

The controller 70 may determine whether or not a progressive-power lens is appropriate for the examinee. For example, the controller 70 may measure the eye position (the position of the eye relative to the spectacle frame) in a distance-examination and in a near-examination first, and then determine whether or not the eye position in each examination and the corridor length of the progressive-power lens are appropriate. For example, the controller 70 may compare the amount of shift of the eye position and the corridor length of the spectacle lens and, if the difference is equal to or greater than a predetermined value, notify the examiner by displaying on, for example, the monitor 4 information to the effect that the progressive-power lens is not appropriate. For example, the distance-examination and the near-examination are switched by changing a convergence angle by the deflection mirror 81 and by changing the target presenting position by the corrective optical system 60.

The controller 70 may measure addition power for a progressive-power lens by causing the corrective optical system 60 to add diopter power to anterior spectacles. For example, the controller 70 may check the vision of the examinee's eye that is wearing the anterior spectacles with minus diopter power, to which the corrective optical system 60 added positive diopter power (addition power), and accordingly, obtains correct addition power.

In the above example, spectacles are described as the refraction corrective device. However, the refraction corrective device may be contact lenses, or a trial frame (trial frame). Also in this case, as in the above description, final prescription values can be determined by making fine adjustments to diopter power during wear of the refraction corrective device.

LIST OF REFERENCE NUMERALS

| 1 | Optometry apparatus |
| 2 | Housing |
| 4 | Monitor |
| 5 | Chin rest |
| 7 | Measuring unit |
| 10 | Objective examination optical system |
| 25 | Subjective examination optical system |
| 30 | Light projecting optical system |
| 45 | First target projecting optical system |
| 46 | Second target projecting optical system |
| 50 | Observing optical system |
| 60 | Corrective optical system |
| 70 | Controller |
| 75 | Memory |
| 90 | Correction optical system |
| 100 | Imaging optical system |

**Claims**

1.  An optometry apparatus that examines the optical property of an examinee's eye, the optometry apparatus comprising:

    a light projecting optical system configured to project target light flux toward the examinee's eye;
    a corrective optical system placed in an optical path of the light projecting optical system, the corrective optical system being configured to change the optical property of the target light flux;
    an information acquisition means configured to acquire information on diopter power of a refraction corrective device worn by the examinee; and
    a control means configured to control the corrective optical system and perform an over-refraction on the examinee's eye during wear of the refraction corrective device;

wherein the control means calculates a prescription value of a new refraction corrective device on the basis of a refractive error acquired by a subjective examination that is performed for the over-refraction, and the diopter power information;

wherein the control means adds the refractive error and the diopter power of the refraction corrective device to calculate the prescription value;

wherein the diopter power information includes the spherical power, cylindrical power, and cylindrical axis of the refraction corrective device;

wherein the refractive error includes a spherical power error, a cylindrical power error, and a cylindrical axis error; and

wherein the control means adds the spherical power error, the cylindrical power error, and the cylindrical axis error respectively to the spherical power, the cylindrical power, and the cylindrical axis to calculate the prescription values.

2. The optometry apparatus according to claim 1, wherein the control means corrects the value of the pupil diameter of the examinee's eye on the basis of the information on the diopter power of the refraction corrective device that is acquired by the information acquisition means.

3. The optometry apparatus according to any of claims 1 or 2, wherein

the refraction corrective device is spectacles, and

the control means outputs information on the fit of the spectacles on the basis of a result of the subjective examination.

4. The optometry apparatus according to any of claims 1 or 2, wherein

the refraction corrective device is spectacles having a pantoscopic angle and a camber angle that are adjusted for the examinee, and

the control means performs an over-refraction on the examinee's eye during wear of the spectacles.

5. The optometry apparatus according to any of claims 1 or 2, further comprising an image acquisition means configured to acquire an image of the face including the anterior segment of the examinee's eye, wherein

the refraction corrective device is spectacles,

the information acquisition means acquires a distance between the optical axes of the spectacles, and

the control means detects a difference between the interpupillary distance of the examinee that is acquired on the basis of the image and the optical axis distance.

**Patentansprüche**

1. Eine Optometrievorrichtung, die die optische Eigenschaft eines Auges einer zu untersuchenden Person untersucht, wobei die Optometrievorrichtung folgende Merkmale aufweist:

ein lichtprojizierendes optisches System, das dazu konfiguriert ist, einen Ziellichtfluss in Richtung des Auges der zu untersuchenden Person zu projizieren;

ein korrigierendes optisches System, das in einem Strahlengang des lichtprojizierenden optischen Systems platziert ist, wobei das korrigierende optische System dazu konfiguriert ist, die optische Eigenschaft des Ziellichtflusses zu ändern;

eine Informationserfassungseinrichtung, die dazu konfiguriert ist, Informationen über die Dioptrienstärke einer von der zu untersuchenden Person getragenen Refraktionskorrekturvorrichtung zu erfassen; und

eine Steuereinrichtung, die dazu konfiguriert ist, das korrigierende optische System zu steuern und eine Überrefraktion an dem Auge der zu untersuchenden Person während des Tragens der Refraktionskorrekturvorrichtung durchzuführen;

wobei die Steuereinrichtung einen Verschreibungswert einer neuen Refraktionskorrekturvorrichtung auf der Basis eines Refraktionsfehlers, der durch eine subjektive Untersuchung erfasst wird, die für die Überrefraktion durchgeführt wird, und der Dioptrienstärkeinformationen berechnet;

wobei die Steuereinrichtung den Refraktionsfehler und die Dioptrienstärke der Refraktionskorrekturvorrichtung addiert, um den Verschreibungswert zu berechnen;

wobei die Dioptrienstärkeinformationen die sphärische Stärke, die zylindrische Stärke und die zylindrische Achse der Refraktionskorrekturvorrichtung umfassen;
wobei der Refraktionsfehler einen sphärischen Stärkefehler, einen zylindrischen Stärkefehler und einen zylindrischen Achsenfehler umfasst; und
wobei die Steuereinrichtung den sphärischen Stärkefehler, den zylindrischen Stärkefehler und den zylindrischen Achsenfehler jeweils zu der sphärischen Stärke, der zylindrischen Stärke und der zylindrischen Achse addiert, um die Verschreibungswerte zu berechnen.

2. Die Optometrievorrichtung gemäß Anspruch 1, wobei die Steuereinrichtung den Wert des Pupillendurchmessers des Auges der zu untersuchenden Person auf der Grundlage der Informationen über die Dioptrienstärke der Refraktionskorrekturvorrichtung korrigiert, die durch die Informationserfassungseinrichtung erfasst werden.

3. Die Optometrievorrichtung gemäß einem der Ansprüche 1 oder 2, wobei

die Refraktionskorrekturvorrichtung eine Brille ist, und
die Steuereinrichtung Informationen über den Sitz der Brille auf der Basis eines Ergebnisses der subjektiven Untersuchung ausgibt.

4. Die Optometrievorrichtung gemäß einem der Ansprüche 1 oder 2, wobei

die Refraktionskorrekturvorrichtung eine Brille mit einem pantoskopischen Winkel und einem Sturzwinkel ist, die für die zu untersuchende Person eingestellt sind, und
die Steuereinrichtung während des Tragens der Brille eine Überrefraktion an dem Auge der zu untersuchenden Person durchführt.

5. Die Optometrievorrichtung gemäß einem der Ansprüche 1 oder 2, die ferner eine Bilderfassungseinrichtung aufweist, die dazu konfiguriert ist, ein Bild des Gesichts einschließlich des vorderen Segments des Auges der zu untersuchenden Person zu erfassen, wobei

die Refraktionskorrekturvorrichtung eine Brille ist,
die Informationserfassungseinrichtung einen Abstand zwischen den optischen Achsen der Brille erfasst, und
die Steuereinrichtung eine Differenz zwischen dem Pupillenabstand der zu untersuchenden Person, der auf der Grundlage des Bildes erfasst wird, und dem optischen Achsenabstand detektiert.

**Revendications**

1. Appareil d'optométrie qui examine la propriété optique d'un œil d'une personne examinée, l'appareil d'optométrie comprenant :

un système optique de projection de lumière configuré pour projeter un flux lumineux cible vers l'œil de la personne examinée ;
un système optique correcteur placé dans un chemin optique du système optique de projection de lumière, le système optique correcteur étant configuré pour changer la propriété optique du flux lumineux cible ;
un moyen d'acquisition d'informations configuré pour acquérir des informations sur une puissance dioptrique d'un dispositif correcteur de réfraction porté par la personne examinée ; et
un moyen de commande configuré pour commander le système optique correcteur et effectuer une sur-réfraction sur l'œil de la personne examinée pendant le port du dispositif correcteur de réfraction ;
dans lequel le moyen de commande calcule une valeur de prescription d'un nouveau dispositif correcteur de réfraction sur la base d'une erreur de réfraction acquise par un examen subjectif qui est effectué pour la sur-réfraction, et des informations de puissance dioptrique ;
dans lequel le moyen de commande additionne l'erreur de réfraction et la puissance dioptrique du dispositif correcteur de réfraction pour calculer la valeur de prescription ;
dans lequel les informations de puissance dioptrique incluent la puissance sphérique, la puissance cylindrique et l'axe cylindrique du dispositif correcteur de réfraction ;
dans lequel l'erreur de réfraction inclut une erreur de puissance sphérique, une erreur de puissance cylindrique et une erreur d'axe cylindrique ; et
dans lequel le moyen de commande ajoute l'erreur de puissance sphérique, l'erreur de puissance cylindrique et l'erreur d'axe cylindrique respectivement à la puissance sphérique, à la puissance cylindrique et à l'axe cylindrique pour calculer les valeurs de prescription.

2. Appareil d'optométrie selon la revendication 1, dans lequel le moyen de commande corrige la valeur du diamètre de la pupille de l'œil de la personne examinée sur la base des informations sur la puissance dioptrique du dispositif correcteur de réfraction qui sont acquises par le moyen d'acquisition d'informations.

3. Appareil d'optométrie selon l'une quelconque des revendications 1 ou 2, dans lequel

   le dispositif correcteur de réfraction est des lunettes, et
   le moyen de commande délivre en sortie des informations sur le positionnement des lunettes sur la base d'un résultat de l'examen subjectif.

4. Appareil d'optométrie selon l'une quelconque des revendications 1 ou 2, dans lequel

   le dispositif correcteur de réfraction est des lunettes présentant un angle pantoscopique et un angle de cambrure qui sont ajustés pour la personne examinée, et
   le moyen de commande effectue une sur-réfraction sur l'œil de la personne examinée pendant le port des lunettes.

5. Appareil d'optométrie selon l'une quelconque des revendications 1 ou 2, comprenant en outre un moyen d'acquisition d'image configuré pour acquérir une image du visage incluant le segment antérieur de l'œil de la personne examinée, dans lequel

   le dispositif correcteur de réfraction est des lunettes,
   le moyen d'acquisition d'informations acquiert une distance entre les axes optiques des lunettes, et
   le moyen de commande détecte une différence entre la distance interpupillaire de la personne examinée qui est acquise sur la base de l'image et la distance d'axe optique.

# FIG. 1

FIG. 2

EP 3 967 211 B1

# FIG. 3

# FIG. 4

FIG. 5

# FIG. 6

# FIG. 7

START

S1 — ANTERIOR SPECTACLE POWER MEASUREMENT

S2 — SUBJECTIVE EXAMINATION DURING WEAR

S3 — SPECTACLE PRESCRIPTION VALUE CALCULATION

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5176893 A
- EP 2949266 A1
- US 2018136486 A1
- JP H1033479 A